# EUROPEAN PATENT APPLICATION

(11) **EP 1 300 673 A1**
(43) Date of publication of application: **09.04.2003**
(21) Application number: 01949504.3
(22) Date of filing: 06.07.2001
(51) Int. Cl.: G01N 21/64, C07K 14/47

(54) **METHOD FOR DETERMINING THE ACTIVITY OF UNCOUPLING PROTEINS (UCPS) BY MONITORING NAD(P)H CONSUMPTION**

(30) Priority: 07.07.2000 ES 200001702
(71) Applicant: CONSEJO SUPERIOR DE INVESTIGACIONES CIENTIFICAS, 28006 Madrid (ES)
(72) Inventor: RIAL ZUECO, Eduardo Ctro.Invest.Biol.Consejo Sup., 28006 Madrid (ES); JIMENEZ JIMENEZ,Jesus Ctro.Inv.Biol., 28006 Madrid (ES)
(74) Representative: Ungria Lopez, Javier
(86) International application number: ES0100273
(87) International publication number: WO02008733

(57) **Abstract**

The present invention concerns the design of a method for determining the activity of UCPs and which method can be used to evaluate the ability of different drugs to modify the activity of said proteins. These drugs may be used in the treatment of all diseases and conditions in which changes in thermogenic activity occur. Examples of these are obesity, fever, cachexia etc.
Since the activity of the UCPs produces changes in the respiration rate, in the present method the activity of the UCPs is determined by monitoring the disappearance of NADH or NAD(P)H from the reduction in absorption between 300 and 380 nm or in fluorescence from 420-520 nm. In this method, these coenzymes are oxidised by the mitochondria of the yeast *Saccharomyces cerevisiae* in which different UCPs are expressed in a recombinant manner.

## Description

### FIELD OF THE TECHNIQUE

Identification of therapeutic compounds. Regulator compounds of the uncoupling proteins (UCPs) of cellular respiration. Therapeutic compounds for the treatment of obesity, non-insulin dependent diabetes, cachexia and fever.

### STATE OF THE ART

Cellular respiration is a process that takes place inside the mitochondria and involves the oxidation of compounds such as sugars or fats. Electron transfer from the molecules that are being oxidised to the oxygen takes place in the internal mitochondrial membrane associated with a proton pump from the inside to the outside of the mitochondria that generates a proton gradient. The energy stored in this gradient will be used for synthesis of ATP in the mitochondria, which is the universal molecule that stores and distributes energy to a large number of processes: manufacturing of cell components, transport, transmission of signals etc. Both processes (respiration and ATP synthesis) constitute so-called oxidative phosphorylation and are perfectly associated in such a way that the rate of oxidation of the substrate (respiration) changes in accordance with the demand the cell makes for ATP synthesis. This coupling process has important consequences for cell economy since energy reserves are not wasted. However, in some physiological situations it is necessary to use up energy. One example is the response in a cold environment to maintain the body temperature. In this case, oxidative phosphorylation is uncoupled so that the energy from cellular respiration can be used to produce heat. This uncoupling is also important to eliminate excess calories ingested with the diet (and, therefore, helps to maintain the body weight) or, for example, to reduce the production of free radicals. Free radicals can damage the cell causing ageing and, eventually, cell death.

Uncoupling proteins (UCPs) belong to a protein superfamily that is comprised of mitochondrial metabolite transporters. Members of this family, that are evolutionarily related, present a series of structural and functional similarities [Ricquier D, Buillard F, (2000) The Uncoupling Protein Homologues. UCP1, UCP2, UCP3, StUCP and AtUCP] Biochem J. 345. 161-179]. The uncoupling protein UCP1, exclusive to the brown fatty tissue of mammals, plays an essential role in maintaining body temperature in the newborn and in small mammals. Similarly, in small mammals this tissue permits excess calories ingested with the diet to be eliminated. The activity of the uncoupling protein UCP1 is not regulated precisely. The hypothalamus emits the signal via the sympathetic nervous system to start thermogenesis releasing noradrenalin. Binding of this hormone to the plasmatic membrane of the adipocyte triggers off, in its interior, a cascade of signals that result in release of fatty acids. These will play a double role: they constitute the substrate to be oxidized by the mitochondria but are also the activators of UCP1. Activation of this protein results in the reintroduction of the protons pumped out by the respiratory chain into the interior of the mitochondria without requiring ATP synthesis. This, therefore, results in an acceleration of respiration and in dissipation of the energy from the proton gradient as heat [Nicholls DG, & Locke RM (1984) Physiol. Rev. 64. 1-64; Cannon B & Nedergaard J (1985) Essays Biochem 20 110-164]. We have recently described that the *todo-trans* retinoic acid is also a potent activator of UCP1 [Rial E. Gonzalez-Barroso MM. *et al*., (1999) Retinoids activate proton transport by the Uncoupling Proteins UCP1 and UCP2 EMBO J 18 5827-5833].

In recent years, a whole series of genes that code for the protein homologues to UCP1 have been discovered. Uncoupling proteins have been discovered in plants (StUCP and AtUCP) that also appear to play a role in resistance to low temperatures. In mammals, the existence of at least three new UCPs (UCP2, UCP3 and UCP4) has been demonstrated although the biological functions and the biochemical activities of these are not yet known. Genetic studies suggest that UCP2 and UCP3 play an important role in energy expenditure in humans. Information about the signals that control gene expression of UCP2 and UCP3 is coming to light but the physiological pathways that regulate their uncoupling activity are, as yet, unknown. We have described that UCP2 is activated by *todo-trans* retinoic acid but not by fatty acids [Rial E. Gonzalez-Barroso MM et al. (1999) Retinoids activate proton transport by the Uncoupling proteins UCP1 and UCP2 EMBO J 18 5827-5833].

Due to the thermogenic potentials of UCP2 and UCP3 and the possibility that their activation can eliminate fat reserves, these have been considered as target cells for obesity treatment and related diseases [Campfield LA., Smith FJ & Burn P (1998) Strategies and potential molecular targets for obesity treatment. Science 280. 1383-1387]. As mentioned previously, *todo-trans* retinoic acid is an activator of UCP2 and is also highly specific for the ligand since only some retinoids can activate the protein. This discovery has resulted in the development of a patent that uses retinoids for the treatment of obesity [Rial E, Gonzalez-Barroso MM, et al. (1999) Retinoids activate protein transport by the uncoupling proteins UCP1 and UCP2. EMBO J 18 5827-5833; Patent ES9800225]. It has also been reported that carboxylic aminoguanidine acid activates thermogenesis in cell lines that express UCP2 and, for this reason, this has been proposed as a drug for the treatment of obesity and diabetes [WO 99/00123, Use of a drug capable of modulating the regulation of UPC-2 and method for screening for potential drugs against obesity].

Most studies on regulation of UCP1 activity, or that of UCP2, determine the bioenergetic properties of the mitochondria by analysing the changes produced in mitochondrial respiration and/or membrane potential. Thus, in the study of UCP1 and UCP2 regulation by retinoids, oxygen consumptions were determined using an oxygen electrode [Rial E, Gonzalez-Barroso MM et *al.,* (1999) Retinoids activate proton transport by the uncoupling proteins UCP1 and UCP2. EMBO J. 18. 5827-5833]. In the study with carboxylic aminoguanidine acid, the activity of UCP-2 was deduced by estimating the rise in the temperature of the cultured cells with an infrared camera [WO 99/00123, Use of a drug capable of modulating the regulation of UCP-2 and method for screening for potential drugs against obesity; WO 99/60630. Infrared thermography]. However, none of these procedures to assess UCP2 activity, or that of other members of this family, can be performed easily, quickly, with a high reproducibility or on a large scale and cannot, therefore, satisfy current needs of large companies that must study chemical libraries with hundreds or thousands of compounds. This approach forms the basis of the development of systems for the identification of new therapeutic compounds, which in this case, for example, can be used to treat obesity and diabetes or to develop new antipyretic drugs. The present invention describes a method that is easy to perform, inexpensive, highly reproducible and capable of processing a large number of samples per time unit.

### DESCRIPTION

### BRIEF DESCRIPTION

The present invention describes a method for determining the activity of uncoupling proteins (UCPs) by monitoring consumption of NAD(P)H and can, therefore, be used to evaluate the ability of different chemical compounds to modify the activity of these proteins. These compounds can be converted into potential drugs against diseases in which variations in thermogenic activity are produced by the activity of these proteins. A reduction of this activity could lead, for example, to obesity or related diseases (diabetes, hypertension etc.). An increase could result in fever or in extreme weight loss such as that occurring in cachexia.

Since variations in the activity of the uncoupling proteins produces changes in the rate of mitochondrial respiration, in the present method the rate of the UCPs is determined by monitoring the disappearance of NADH or NAD(P)H. The measuring method uses absorption of light in the region between 300 and 380nm and/or of the fluorescence emitted between 420 and 520 nm by NAD(P)H. These compounds are the substrate that is oxidised by mitochondria of the yeast *Saccharomyces cerevisiae* used in the present invention that have been genetically transformed so that they express the recombinant form of UCP1. The following, therefore, form part of the invention:
- this identification method in which the transformed cells from which the mitochondria are obtained are competent cells as defined in the present invention.
- this identification method in which the uncoupling protein is not UCP1 and belongs, among others, to the following group: UCP2, UCP3, UCP4, StUCP and AtUCP.
- the new compounds identified as regulators of the activity of uncoupling proteins UCPs, whether they be inducers or inhibitors,
- and the use of these to treat diseases in which the activity of these uncoupling proteins is altered.

### DETAILLED DESCRIPTION

As described previously, the UCPs can be expressed in the strain W303 of *Saccharomyces cerevisiae* using the vector pYeDP/-1/8-10 in which the expression of the protein is under control of the gal-cyc promoter [Cullin C, Pompon D (1988) Synthesis of functional mouse cytochromes P450 P1 and chimeric P-450 P3-1 in the yeast *Saccharomyces cerevisiae* Gene 65: 203-217]. With this system, expression is repressed in the presence of glucose and is activated with galactose. The present invention describes a method to assess the activity of these uncoupling proteins using UCP1 as an example of a UCP that is stably expressed in yeasts and from which the mitochondria required for this assessment can be later obtained. Therefore, strains of recombinant *Saccharomyces cerevisae* have been constructed capable of expressing the uncoupling protein UCP1.

Details of the materials and procedures used to construct the expression vector (pYeDP-UCP), transformation of the strain *Saccharomyces cerevisiae* W303 and selection of the clones that express UCP1 have been published previously [Arechaga I, Raimbault S, et al., (1993) Cysteine residues are not essential for uncoupling protein function. Biochem J 296: 693-700]. A similar methodology has been used to construct strains that express UCP2 or UCP3 [Fleury C, Neverova M, *et al.,* (1997) Uncoupling protein 2 a novel gene linked to obesity and hyperinsulinemia. Nature Genet. 15: 269-272, Zhang CY, Hagen T, *et al*., (1999) assessment of uncoupling activity of uncoupling protein 3 using a yeast heterologous expression system FEBS Lett 449 129-134]. The use of any other uncoupling protein [Ricquier D, Bouillard F. (2000) The uncoupling protein homologues UCP1, UCP2, UCP3, StUCP and AtUCP Biochem J. 345 161-179] different from the UCP1 used as an example of the method of the present invention is also possible and the most interesting of these, among other possible options, are the proteins UCP2, UCP3, UCP4, StUCP and AtUCP and forms part of the present invention. On the other hand, there are other alternative expression vectors in yeasts [Goeddel DV (editor)(1990) Gene Expression Technology Methods Enzymol. Vol. 185], that permit controlled expression of these uncoupling proteins in the method described in the present invention and form part of the present invention. As a control for the studies using yeast mitochondria that express UCPs the same yeast strain has been used but this has been transformed with a vector pYeDP in which the cDNA clone of UCP1 is inserted in the opposite sense (producing an anti-sense mRNA from which protein cannot be produced) [Arechaga I, Raimbault S *et al.*, (1993) Cysteine residues are not essential for uncoupling protein function. Biochem. J. 296 693-700; Rial E. Gonzalez Barroso MM, *et al.,* (1999) Retinoids activate proton transport by the uncoupling proteins UCP1 and UCP2 EMBO J. 18: 5827-5833. Patent ES9800225].

To study uncoupling proteins, procedures have been developed for the expression of different uncoupling proteins in yeasts, in particular in *Saccharomyces cerevisiae.* The main advantage of using these yeasts is that this is a eukaryotic microorganism and its mitochondria can incorporate the UCPs in a totally functional manner [Arechaga I, Raimbault S *et al*, (1993) Cysteine residues are not essential for uncoupling protein function. Biochem. J. 296:693-700]. On the other hand, yeast cultures are inexpensive, and large amounts of biological material can be obtained. Moreover, the mitochondria of *S. cerevisiae* have one quality that makes them particularly suitable for the present method since they can oxidise NAD(P)H added to the growth medium. These microorganisms (*S. cerevisiae*) have a dehydrogenase, which accepts the substrate, NAD(P)H, on the external surface of the internal mitochondrial membrane. This property also appears in other yeasts such as *Candida utilis* or *Saccharomyces carlsbergensis* [Van Dijken JP. Scheffers WA (1986) Redox balances in the metabolism of sugars by yeasts. FEMS Microbiol Rev. 32: 199-224] and contrasts with findings in mammalian mitochondria. In the present invention, competent cells are those cells, whether they be yeast cells or not, whose mitochondria have the ability, natural or artificially acquired, to oxidize NAD(P)H added to the medium thanks to the existence of a dehydrogenase that accepts this substrate on the external surface of the internal membrane of the mitochondria. The present invention describes the use of the yeast *Saccharomyces cerevisiae* as a model for expression of these uncoupling proteins and the obtention of mitochondria suitable for performing the method of the invention. *Saccharomyces cerevisae* is an example of a competent cell, and the use of any of these in the method of the present invention forms part of the present invention.

Subsequently, mitochondria from *Saccharomyces cerevisiae* transformed with UCP1 were isolated and prepared for use in assays to evaluate regulatory compounds of the activity of this UCP (Example 2).

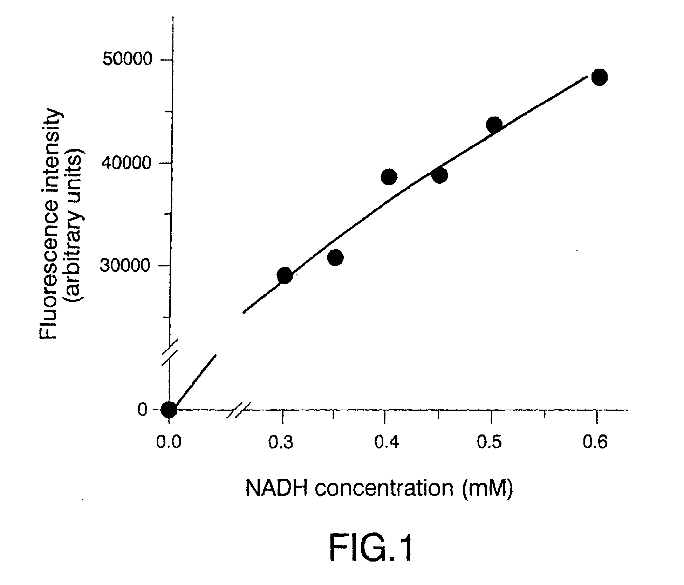

The method for determining the activity of the uncoupling proteins (UCPs) described in this invention is based on the fact that the activity of uncoupling proteins produces changes in the rate of mitochondrial respiration and that this can be evaluated, parallely, by monitoring the disappearance of NADH or NAD(P)H (substrates for respiration). Addition of these substrates followed by their disappearance (as a consequence of their oxidation in the mitochondria) can be perfectly controlled in the method described in the present invention (Example 1). NADH and NADPH present two absorption bands in the ultraviolet region. The first has a peak at 260 nm and the second at 340nm. In the oxidised form of both coenzymes the 340nm band disappears. The light absorbed in this second region of the spectrum (between 300 and 380 nm) is emitted as fluorescent light between 420 and 520 nm (emission peak at 460 nm). Therefore, the changes in absorption of the 300-380nm band or the change in fluorescence in the region from 420-520nm can be used to monitor consumption of NAD(P)H. These levels of NAD(P)H fluorescence or absorption that, therefore, correspond to the remaining concentration of these substrates can be represented graphically (Figure 1) and permit the changes in NAD(P)H concentration with time to be monitored in a solution. Figure 1 shows the fluorescence signal measured with a POLAR star Galaxy plate reader (BMG Laboratories Gmbh, Offenburg, Germany) using a filter for excitation at 340 nm and one for emission at 460 nm. A total of 200µl of a control suspension of *S. cerevisiae* mitochondria is introduced to the plate wells (0.1 mg protein/ml) and increasing concentrations of NADH (0.3 to 0.6 mM). The mathematical equation that relates fluorescence with concentration is used in subsequent examples to convert the fluorescence signal into NADH concentration.

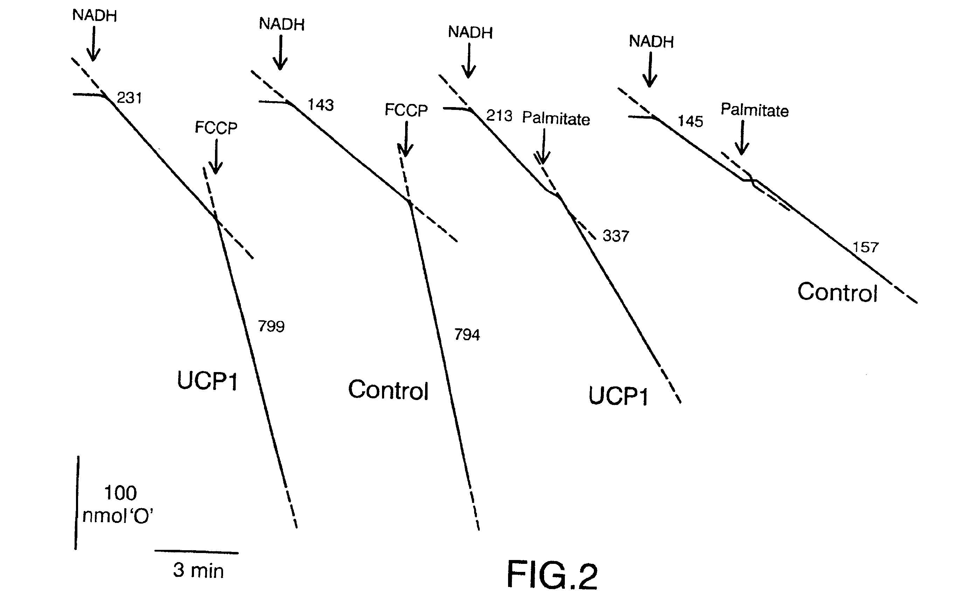

Figure 2 shows a classical example of the determination of oxygen consumption in Control yeasts and in yeasts which express UCP1 following the methodology described previously [Arechaga I, Raimbult S. et al., (1993) Cysteine residues are not essential for uncoupling protein function. Biochem J. 296: 693-700]. The basal respiration rate is shown (stage 4) after NADH addition and after addition of the uncoupling agent FCCP (Trifluoromethoxy-carbonylcyanide phenylhydrazone) at a concentration of 10µM that induces a maximum respiration rate in the mitochondria. The basal respiration of UCP1 mitochondria is greater than that of the Control because, in the absence of inhibitor, UCP1 is partially activated. The maximum respiration rate (in the presence of FFCP) is similar in both preparations indicating that the respiratory potential of the mitochondria of the two strains of yeasts (UCP1 and Control) is the same. Palmitate stimulates the respiration more in UCP1 mitochondria than in Control mitochondria revealing its specific activation [Arechaga I., Raimbult S. *et al*., (1993) Cysteine residues are not essential for uncoupling protein function. Biochem. J. 296: 693-700]. Respiration rates are shown next to the electrode readings and in Table 1. This example represents a model of the responses expected in the group of Control yeasts and in those that express UCP1 and permits the results obtained by the new method described in the present invention as indicated in the following:

**Table 1.**

| **Comparison of the respiration rates estimated from oxygen consumption determinations with the values obtained from NADH consumption measurements.** | | |
|---|---|---|
| **CONTROL mitochondria** | **Oxygen consumption**^{**1**} | **NADH consumption**^{**2**} |
| **Basal respiration** | **144** | **141** |
| **Uncoupled respiration** | **763** | **691** |
| **GDP 3mM** | **155** | **151** |
| **Palmitate 48µM** | **157** | **148** |
| **Palmitate-GDP** | **157** | **157** |
| | | |
| **UCP1 mitochondria** | **Oxygen consumption**^{**1**} | **NADH consumption**^{**2**} |
| **Basal respiration** | **218** | **223** |
| **Uncoupled respiration** | **773** | **675** |
| **GDP 3 mM** | **149** | **142** |
| **Palmitate 48µM** | **337** | **349** |
| **Palmitate + GDP** | **140** | **147** |

| | | |
|---|---|---|
| ^{**1**}**Values in nmoles of oxygen consumed per minute and per mg of protein.** | | |
| ^{**2**}**Values in nmoles of NADH consumed per minute and per mg of protein.** | | |

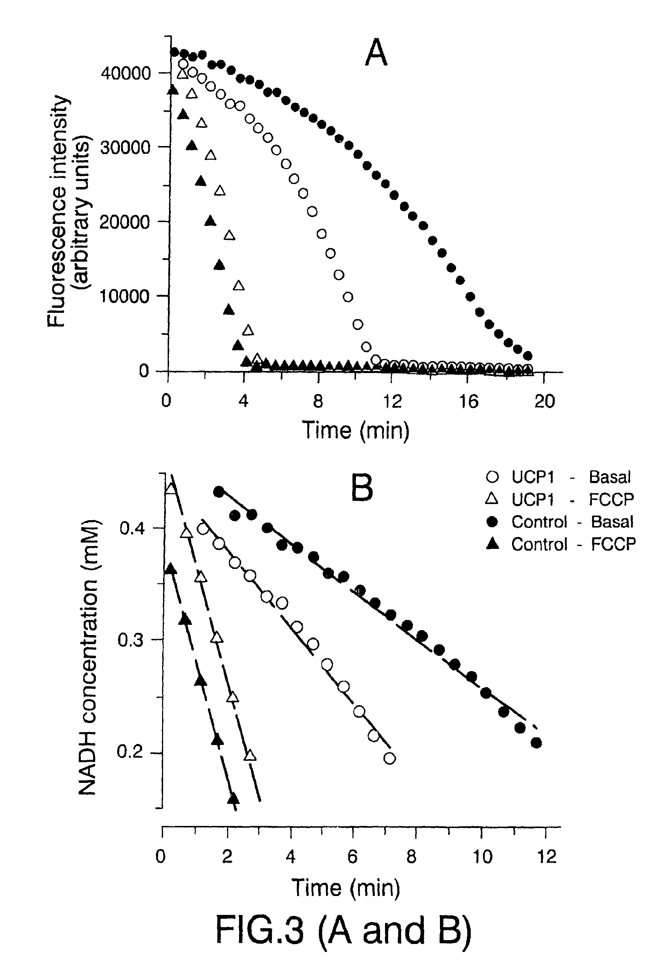

Figure 3 shows a parallel experiment to the previous one in which the respiration rate is estimated from the values of NADH consumption measured with a POLAR star Galaxy plate reader as in Figure 1. Figure 3A shows the curves of NADH fluorescence reduction for Control mitochondria and UCP1 both in the basal state and in the presence of FCCP. The results obtained with this method are identical to those obtained in Figure 2 using the oxygen electrode (see Table 1), i.e. a greater basal respiration in UCP1 than in the Control and comparable rates in the presence of the uncoupling FCCP. Figure 3B shows the conversion of the fluorescence signal to NADH concentration using the equation obtained in Figure 1 using only values in the 0.2-0.6 mM NADH range, which is where the most reliable determinations are made. Rates are calculated from the gradient of the regression line and are shown in Table 1 where the values obtained are compared with values of oxygen consumption.

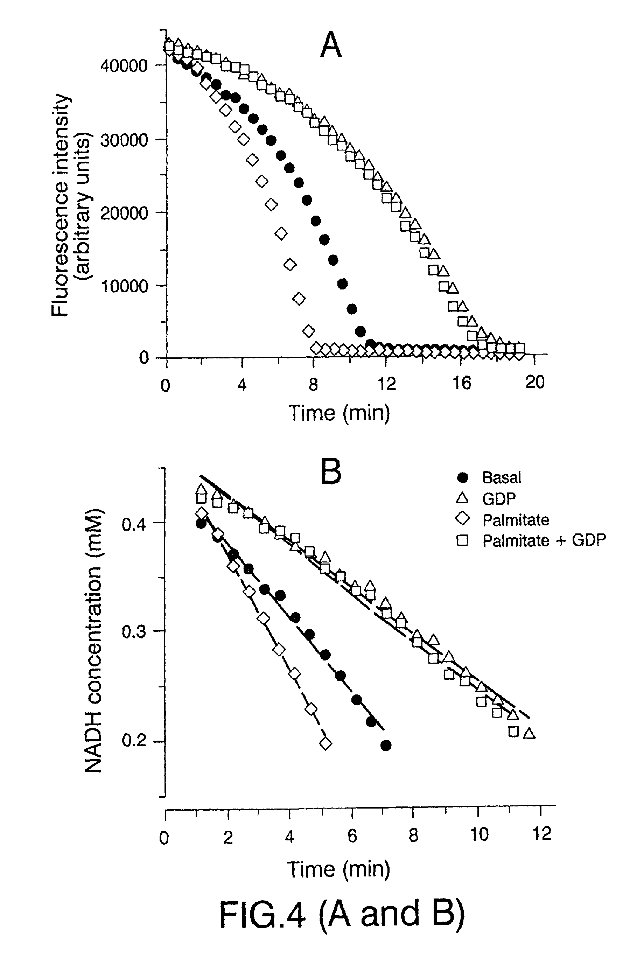

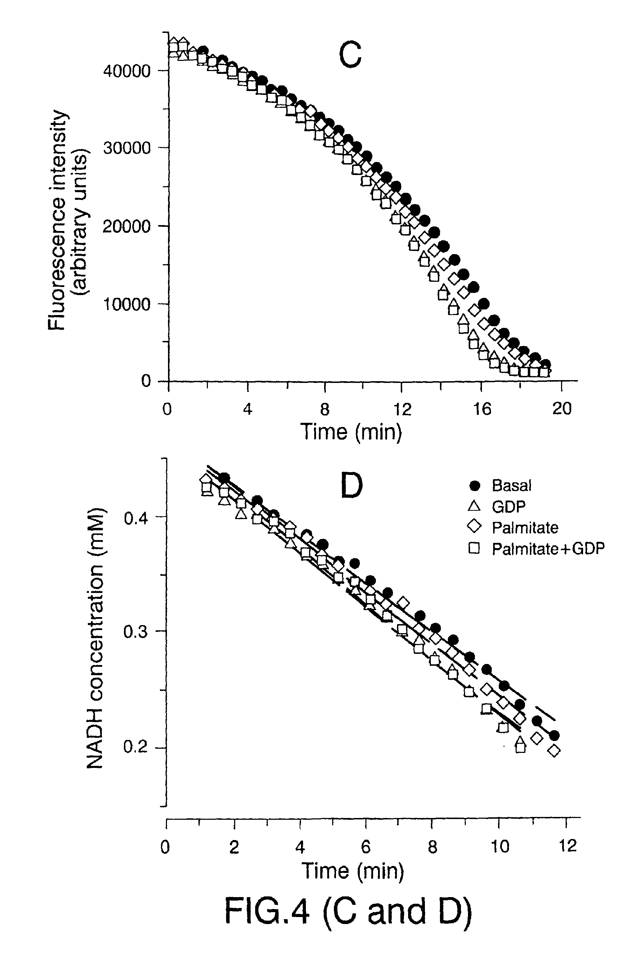

Figure 4 shows the effect of an activator (palmitate) and that of an inhibitor (GDP) of UCP1 on the NADH consumption rate, both compounds are known regulators of uncoupling proteins (Example 2)[Rial E. Gonzalez-Barroso MM. *et al*., (1999) Retinoids activate proton transport by the uncoupling proteins UCP1 and UCP2. EMBO J. 18. 5827-5833, Arechaga I, Raimbault S. *et al*., (1993) Cysteine residues are not essential for uncoupling protein function. Biochem. J. 296 693-700]. Graphs 4A and 4C show the reduction in fluorescence for the UCP1 mitochondria (Fig. 4A) and the Control mitochondria (Fig. 4C). The conversion to NADH concentrations is shown in Graphs 4B and 4D and, as with Figure 3, only the interval in which the most reliable determinations are obtained is taken into account. The rates of NADH consumption calculated from the corresponding regression lines are shown in Table 1. As expected, the rate of disappearance of NADH from the UCP1 mitochondria is greater in the presence of the activator (palmitate, rhombi) and less when inhibitor is added (GDP, triangles) relative to basal respiration levels (circles)(349 and 142, respectively *vs* 223)(Table 1). The results obtained with palmitate and GDP in Control mitochondria are also shown and demonstrate the specificity of the effect of these two regulators for mitochondria that express UCP1 since no differences are observed between the values obtained (148 and 151 respectively *vs* 141). The effects of these two compounds on UCP1 have already been described and previously studied in the literature [Arechaga I., Raimbult S. et *al*.,(1993) Cysteine residues are not essential for uncoupling protein function. Biochem J. 296: 693-700] and correspond with those obtained in the present invention. Example 2 of the present invention is descriptive of the new method for the identification of regulatory compounds of the activity of uncoupling proteins and forms part of the present invention. Hence, inducers of the activity of uncoupling proteins will behave similarly to palmitate, i.e. a rise in NADH consumption will be observed, whereas compounds that inhibit the activity of the uncoupling proteins will behave similarly to GDP, i.e. a reduction in NADH consumption will be observed. All these new compounds that are regulators of protein uncoupling activity, whether they be inhibitors or inducers, identified by the method described in the present invention, form part of the present invention. It is noteworthy that the method of this invention is easier to perform, quicker and easier to reproduce than procedures used to date (determination of oxygen consumption using an oxygen electrode or an infrared camera), which leads us to suggest that the method of the present invention is a valuable new alternative for the identification of regulators of protein uncoupling activity. These potential compounds identified by the method described in the present invention as regulators of uncoupling protein activity or as regulators of oxygen consumption are potential therapeutic compounds for the treatment of diseases in which the activity of these proteins is altered. Thus, the inducers of protein uncoupling activity are therapeutic compounds for use in diseases such as diabetes, non-insulin dependent diabetes and metabolic syndromes and those in which there is a rise in the production of free radicals such as hypoxia, whereas compounds that act as inhibitors of uncoupling protein activity are therapeutic compounds for conditions such as cachexia and fever. All the therapeutic uses of these compounds form part of the present invention.

Table 1 shows the respiration rates obtained with the oxygen electrode and with NADH fluorescence. Values have been obtained for both techniques using the same mitochondrial preparations.

In the examples of the present invention, NADH has been used as a model substrate, the presence of which can be evaluated throughout respiration by determining the fluorescence intensity. As mentioned previously, in the present invention the substrate NADPH presents the same characteristics as NADH and, therefore, the use of NADPH in the method of the present invention forms part of the present invention. On the other hand, monitorization of NADH levels in the present invention has been done by determining fluorescence but this can also be done by determining absorbance, that also forms part of the present invention.

### DESCRIPTION OF THE FIGURES

**Figure 1. Measurement of the fluorescence of NADH.** The fluorescence intensity at 460 nm (excitation at 340 nm) relative to increasing concentrations of NADH is shown.
**Figure 2. Oxygen consumption in mitochondria that express UCP1 and in Control mitochondria.** A comparison between basal respiration and respiration in the presence of uncoupling agent (10µM FCCP) and when stimulated with 48µM palmitate. The dotted lines are the lines used to calculate the gradients in the different regions of the graphs and the values in nmoles of oxygen consumed per minute and miligrams of protein are indicated in the same area of the graph.
**Figure 3. Consumption of NADH by mitochondria that express UCP1 (empty symbols) and control mitochondria (black symbols).** Comparison between basal respiration (circles) and respiration in the presence of 10 µM FCCP (triangles). A. Fluorescence signal; B, conversion to NADH concentration from the titres recorded in Figure 1.
**Figure 4. Changes in the rate of NADH consumption in the presence of activators and inhibitors of uncoupling protein UCP1.** Figure 4A and 4B, UCP1 mitochondria: Figure 4C and 4D control mitochondria. A and C, Fluorescence signal; C and D, conversion to NADH concentration from the titres in Figure 1. The consumption of NADH is compared in basal conditions (circles), with the consumption in the presence of 3 mM GDP to inhibit UCP1 (triangles), the consumption in the presence of 48µM palmitate to activate UCP1 (rhombi) and the consumption in the presence of the two agents, palmitate and GDP (squares).

### EXAMPLES

### Example 1.-Determination of uncoupling activity of the protein UCP1

### Example 1.1.-Preparation of S. cerevisae mitochondria.

To isolate the mitochondria required for the subsequent experiments, yeasts of the strain *Saccharomyces cerevisiae* W303 that express UCP1 were inoculated into SP growth medium (0.67% nitrogen substrate, 0.1% casamino acids, 20 mg/ml tryptophan, 40 mg/ml adenine, 0.1% phosphate, 0.12% ammonium sulphate, 0.1% glucose, 2% lactic acid, pH 4.5) 36 hours before extraction of the mitochondria; 12 or 14 hours before extraction they were diluted in SG medium (0.67% nitrogen substrate, 0.1% casamino acids, 20 mg/ml tryptophan, 40 mg/ml adenine, 2% galactose pH4.5) adjusting the optical density at 600nm to between 0.3-0.4 absorbance units [Arechaga I, Raimbault S. *et al.,* (1993) Cysteine residues are not essential for uncoupling protein function. Biochem. J. 296. 693-700].

The mitochondria were prepared following a procedure based on that of Guerin *et al.*, [Guerin B, Labbe P. Somlo M (1979) Preparation of yeast mitochondria (*Saccharomyces cerevisiae*) with good P/O and respiratory ratios Methods Enzymol. 55: 149-159]. The protoplasts are prepared by enzymatic digestion of the cell wall with cytohelicase and after their homogenization mitochondria are isolated by differential centrifugation [Arechaga I., Raimbault S. *et al.,* (1993) Cysteine residues are not essential for uncoupling protein function. Biochem. J. 296. 693-700]. The same method was used for isolation of yeast mitochondria that express UCP2 or UCP3 [Rial E., Gonzalez-Barroso MM. *et al.,* (1999) Retinoids activate proton transport by the uncoupling proteins UCP1 and UCP2 EMBO J 18. 5827-5833].

### Example 1.2-Determination of the activity of protein UCP1.

Two methods are used to determine the activity of UCP1 in the present invention.

### Example 1.2.1.-Determination of the respiration rate in mitochondria that express UCP1 using an oxygen electrode.

The first method has been described previously and is the one usually used. This method is based on determination of the respiration rate from the oxygen consumption [Arechaga I. Raimbault S. *et al.,* (1993) Cysteine residues are not essential for uncoupling protein function. Biochem. J. 296 693-700; Patent ES9800225]. The mitochondria (protein concentration 0.15 mg/ml) are placed in the oxygen electrode chamber (a total volume of 1ml)(HANSATECH. Kings Lynn, England) with a growth medium that contains 0.65M mannitol, 0.5 mM EGTA, 20 mM Tris/maleate, 10mM phosphate, 2 mM magnesium chloride, pH 6.8. Assays were performed at 20ºC, therefore, the concentration of oxygen in the medium is considered to be 284 nmol/ml. 1 mg/ml of bovine seroalbumin was also added (free from fatty acids). Respiration was initiated with the addition of 1mM NADH. This methodology has also been used to determine the respiratory activity in mitochondria that express UCP2 or UCP3 [Rial E. Gonzalez-Barroso MM. et al., (1999) Retinoids activate proton transport by the uncoupling proteins UCP1 and UCP2. EMBO J. 18: 5827-5833]. The results are represented in Figure 2 and the values in Table 1.

### Example 1.2.2.-Determination of the respiration rate in mitochondria that express UCP1 by monitoring NADH fluorescence.

The second method, which is the object of the present invention, is based on determining the respiration rate by measuring the consumption of NAD(P)H. All the assays were performed on a 96-well micro-titre standardized plate (Nunc., Denmark). The growth medium and other conditions are the same as those described for the oxygen electrode assays except the total volume in each test that is 200µl per well and the substrate concentration. The concentration of NAD(P)H has been reduced to 0.5 mM to increase the sensitivity of the test. 10µl of a solution of 10 mM NADH was added to each well by an injector that forms part of the measuring equipment. Fluorescence measurements were done using a POLAR star Galaxy plate reader (BMG Laboratories OmbH, Offenburg, Germany). The excitation wavelength was 340nm and the emission wavelength was 460nm. Changes in the fluorescence signal were determined every 30 seconds in each well with an average of 6-9 readings for each measurement. The plate was automatically shaken at the end of each cycle for 10 seconds to ensure that the mitochondria remained in suspension. Incubation conditions resulted in at least 6 sites of the wells with maximum respiration rates (in the presence of 10µM of uncoupling FCCP). For the experiments shown in Figure 1,3 and 4 measurements are done in triplicate (3 wells of the plate with the same conditions) and averages were taken of the reduced fluorescence measurements. From these values, fluorescence values for the suspension of mitochondria in the absence of NADH *(blank)* were subtracted.

Conversion of fluorescence values to NADH concentrations (after calculating the average of the values in triplicate and subtracting the *blank* value) is done by first titrating the fluorescence intensity against increasing concentrations of NADH. For these determinations, NADH concentrations of between 0.2 and 0.6mM are used and the assay conditions are identical to those described in the previous paragraph.

### Example 2.-Characterisation of regulators of UCP1.

Regulator compounds of the uncoupling proteins UCP1 and UCP2 are already known. In the presence of these regulators the rate of mitochondrial respiration varies [Rial E., Gonzalez-Barroso MM. *et al*., (1999) Retinoids activate proton transport by the uncoupling proteins UCP1 and UCP2. EMBO J. 18 5827-5833]. Hence, purine nucleotides such as GDP are inhibitors of UCP1 and, therefore, addition of this regulator causes a reduction in the respiration rate [Arechaga I, Raimbault S. et al., (1993) Cysteine residues are not essential for uncoupling protein function. Biochem. J. 296: 693-700]. In the case that an activator is added to the growth medium, such as fatty acids, the respiration rate increases. Therefore, to determine whether a compound is a regulator of the activity of an uncoupling protein, monitorization of the respiration rate permits the nature of the action to be determined. The comparison of its effect with the effect that takes place in Control mitochondria without UCPs permits one to establish whether the effects are specific or not [Rial E, Gonzalez-Barroso MM., *et al.,* (1999) Retinoids activate proton transport by the uncoupling proteins UCP1 and UCP2 EMBO J. 18 5827-5833. Patent ES9800225].

In the present example, the effect of an inducer (palmitate) and an inhibitor (GDP) of uncoupling proteins are determined to validate the method described in the present invention to identify compounds capable of regulating the activity of these uncoupling proteins. The determination was done in triplicate in the same conditions as those described in Example 1.2.2. except that, before addition of NADH, 3 mM of GDP was added to the microtitre wells or 48µM of palmitate or a mixture of 48µM of palmitate and 3 mM GDP (Figure 4). To some of the wells on the same plate, mitochondria without regulators have been added (basal respiration) and to other wells 10µM FCCP (uncoupled respiration). The straight lines in panels B and D are obtained by linear regression of the data shown. The gradients of the regression lines, converted to values of NADH consumption per minute and milligrams of protein are compiled in Table 1.

In the UCP1 mitochondria, NADH consumption is reduced compared to basal levels when the GDP inhibitor is present (triangles), even when this is simultaneously present with palmitate (squares). The activator palmitate increases the rate of NADH consumption (rhombi). In the control mitochondria, these regulators do not produce significant changes in the rate of NADH consumption.

## Claims

1. Method of identification of compounds that regulate the activity of uncoupling proteins of cellular respiration **characterised in that** it comprises the following stages:
Incubation of mitochondria with the compound to be tested in a solution that contains the following coenzymes: NADH or NADPH,
Determination of the rate of disappearance of the coenzyme NADH or NADPH and
Identification of a chemical compound as an inducer of the activity of uncoupling proteins when a rate of disappearance of the coenzyme greater than that observed in the control group is determined, or as an inhibitor of the activity of uncoupling proteins when the rate of disappearance of the coenzyme is less than that observed for a control group.

2. Method according to claim 1 **characterised in that** the uncoupling proteins belong to the following group, among others: UCP1, UCP2, UCP3, UCP4, StUCP and AtUCP.

3. Method according to one of claims 1 to 2 **characterised in that** the mitochondria come from transformed cells that have the ability to express any of the uncoupling proteins of claim 2.

4. Method according to claim 3 **characterised in that** the cells are competent since their mitochondria are capable of oxidising NADH or NADPH.

5. Method according to claim 4 **characterised in that** the competent cells are yeasts.

6. Method according to Claim 4 **characterised in that** the competent cells are the yeasts *Saccharomyces cerevisiae*

7. Method according to any of the claims 1 to 6 **characterised in that** the rate of disappearance of the coenzymes NADH or NADPH are determined, by the following techniques, among others: fluorescence or absorption.

8. Use of the compounds identified according to any of the claims 1 to 7 as regulators of the activity of uncoupling proteins.

9. Use of compounds identified according to claim 8 as inducers in the treatment of pathologies associated with a decrease of the activity of uncoupling proteins belonging to the following group, among others: obesity, non-insulin dependent diabetes and metabolic syndrome.

10. Use of the compounds identified according to claim 8 as inhibitors in the treatment of diseases associated with a rise in the activity of uncoupling proteins belonging to the following group, among others: cachexia and conditions such as fever.

11. Use of the compounds identified according to claim 8 as inducers of the activity of uncoupling proteins in the treatment of conditions in which a rise in the production of free radicals occurs, such as hypoxia.

12. Use of compounds identified according to claim 8 as regulators of consumption of cellular oxygen that modulate the mitochondrial respiratory activity.
